# EUROPEAN PATENT SPECIFICATION

(11) **EP 0 451 612 B1**
(45) Date of publication and mention of the grant of the patent: **02.01.1997**
(21) Application number: 91104815.5
(22) Date of filing: 26.03.1991
(51) Int. Cl.: C12P 21/08, C12N 15/06, G01N 33/577, A61K 39/395

(54) **Anti-oncostatin M monoclonal antibodies**
Anti-Oncostatin-M monoklonale Antikörper
Anticorps monoclonaux anti-oncostatine M

(30) Priority: 29.03.1990 US 501824; 04.03.1991 US 664191
(43) Date of publication of application: 16.10.1991
(73) Proprietor: Bristol-Myers Squibb Company, New York, N.Y. 10154 (US)
(72) Inventor: Radka, Susan F., Seattle, Washington 98103 (US); Linsley, Peter S., Seattle, Washington 98119 (US); Shoyab, Mohammed, Seattle, Washington 98119 (US)
(74) Representative: Kinzebach, Werner, Dr.

(56) References cited:
- GB-A- 2 185 485

## Description

The present invention relates to anti-Oncostatin M monoclonal antibodies. The antibodies of the invention are characterized as being capable of binding to Oncostatin M, inhibiting Oncostatin M receptor binding and/or inhibiting Oncostatin M bioactivity. The monoclonal antibodies of the present invention may be used to detect the presence of Oncostatin M and/or to modulate Oncostatin M biological activities in an in vivo or in vitro system.

It is now well recognized that somatic cell hybrids ("hybrids") are an important source of specific cellular products that cannot be obtained from short-term primary cultures. The best example of this is the system developed by Milstein and others for the production of hybrid myelomas which make a monoclonal antibody against an antigen of choice (Kohler and Milstein, 1985, Nature (London) 256:495; Galfre et al., 1977, Nature (London) 266:550). Such hybrids provide a constant supply of monoclonal antibody against specific antigens. These antibodies can be used as reagents for any procedure for which antibodies were previously used, but with the added advantage of higher levels of discrimination, lower background and a continuous available supply of the antibodies.

The production of monoclonal antibodies in general first involves immunization, removal of immune response cells, fusion of these cells in, for example, polyethylene glycol, with constantly dividing tumor cells ("immortal") selected for their inability to secrete an immunoglobin. The resulting cells (hybridomas) are distinguished by growth in, for example, HAT (hypoxanthine, aminopterin, thymidine) medium. Each hybridoma is the fusion product of a single antibody-forming cell and a tumor cell. The hybridoma has the ability of the former to secrete a single species of antibody and the immortality of the latter, enabling it to proliferate continuously, thus providing a cell progeny which produces an unending supply of antibody with a single specificity.

GB 2 185 485 discloses the cell growth regulatory factor Oncostatin M. This document also makes reference to monoclonal antibodies specific for Oncostatin M.

The present invention involves the production and use of monoclonal antibodies specific for Oncostatin M, a novel cytokine which exhibits pleiotropic effects on a wide variety of normal and transformed cells.

In particular, the present invention refers to a monoclonal antibody, or fragment thereof, the antigen binding site of which binds to Oncostatin M and competitively inhibits the immunospecific binding of monoclonal antibody OM2, OM3, or OM1 produced by hybridoma cell lines 11R2F8, 12R13D7 and 1R10F11, respectively, to its target antigen.

Any monoclonal antibody having the characteristics of the monoclonal antibodies described herein are within the scope of the present invention. For example, a monoclonal or chimeric antibody which competitively inhibits the immunospecific binding of the monoclonal antibodies described herein to their Oncostatin M epitopes and/or which modulate Oncostatin M biological activities are within the scope of the invention. The antibodies of the present invention may bind to the active site of Oncostatin M. In particular, the antibodies may inhibit the biological activity of Oncostatin M.

The invention is described by way of examples in which hybridoma technology is used to generate the anti-Oncostatin M antibodies of the invention, but the scope of the invention is not intended to be restricted to the use of such cell hybridization techniques. The exemplary antibodies are grouped according to whether they form immunoprecipitates with either native Oncostatin M, denatured Oncostatin M, or both. Each group is characterized further by the ability to block Oncostatin M mediated growth inhibition and/or binding of Oncostatin M to its cell surface receptors. Such antibodies are utilized to map epitopes and functional sites of novel Oncostatin M proteins.

The following terms, as used, have the indicated meanings:
- DDEIA --: double determinant enzyme-linked immunoassay
- GIA --: growth inhibitory assay
- HRP --: horseradish peroxidase
- micro-EIA --: micro-enzyme linked immunoassay
- MAb --: monoclonal antibody
- OM --: Oncostatin M
- RRA --: radioreceptor assay
- OM1 --: 1R10F11 monoclonal antibody
- OM2 --: 11R2F8 monoclonal antibody
- OM3 --: 12R13D7 monoclonal antibody
- OM4 --: 4R12C7 monoclonal antibody
- OM5 --: 3R9D9 monoclonal antibody
- OM6 --: 3R13F4 monoclonal antibody
- OM7 --: 12R13B5 monoclonal antibody
- OM8 --: 12R19E3 monoclonal antibody

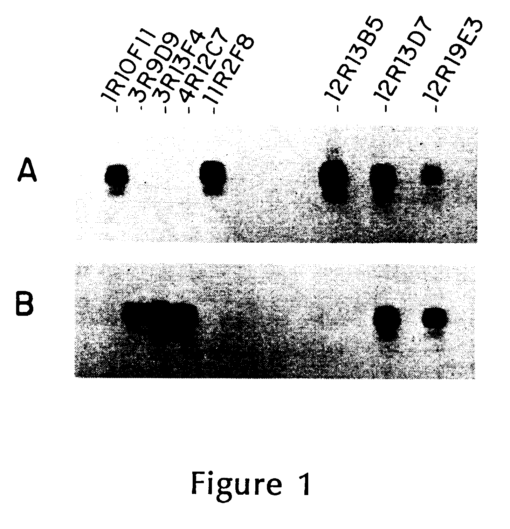

Figure 1 shows the immunoprecipitation of ³⁵S-methionine and ³⁵S-cysteine labelled Oncostatin M from supernatants of the CHO cell line stably transfected with cDNA encoding Oncostatin M. Figure 1A shows the reactivity of a series of anti-Oncostatin M monoclonal antibodies with "native" metabolically labelled Oncostatin M (supernatant collected from metabolically labelled CHO transfectants). Figure 1B shows the reactivity of these same antibodies with supernatant, collected from metabolically labelled CHO cells, which was denatured by treatment with SDS, 2-mercaptoethanol, and boiling prior to incubation with the monoclonal antibodies. Lane 1: negative control antibody; lane 2: OM1; lane 3: OM5; lane 4: OM6; lane 5: OM4; lane 6: OM2; lane 7: OM7; lane 8: OM3; lane 9: OM8.

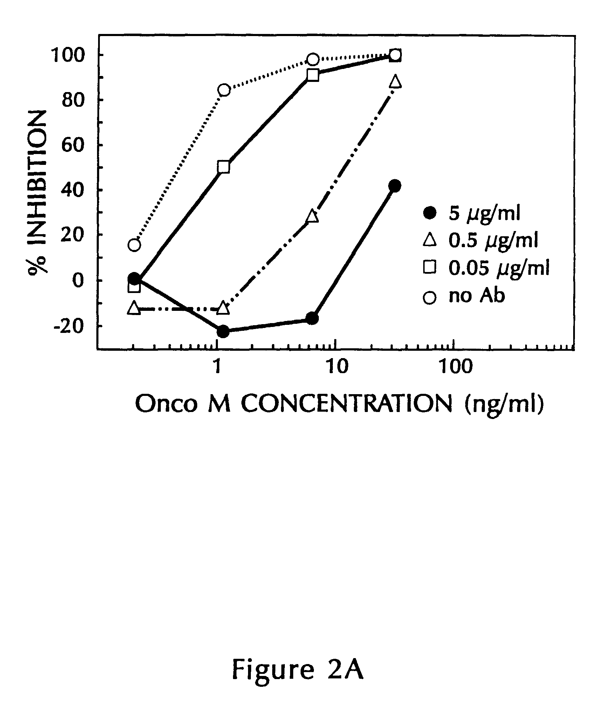

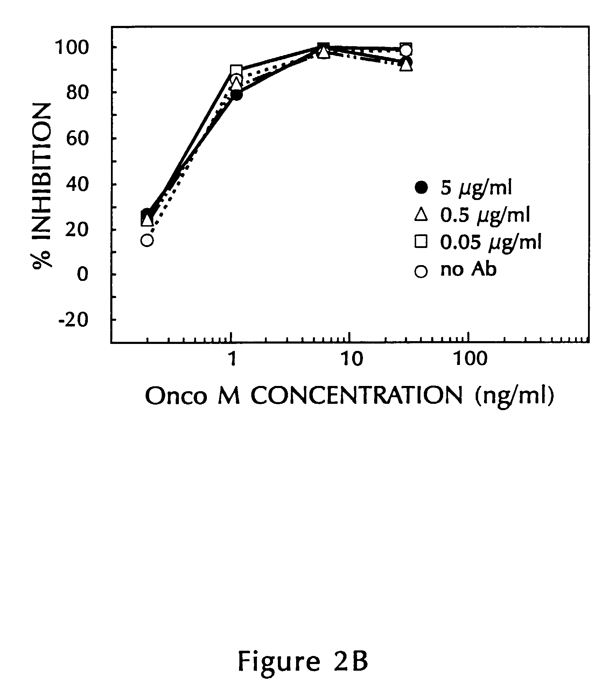

Figure 2 provides data from the examination of neutralizing activity of two different anti-Oncostatin M monoclonal antibodies in an GIA on the A375 melanoma cell line. Figure 2A shows the activity of various concentrations of OM2 and Figure 2B shows the activity of various concentrations of OM1.

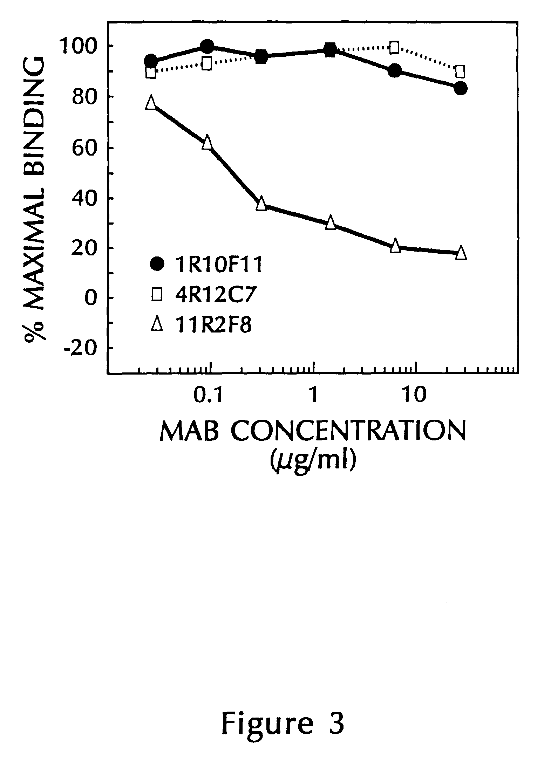

Figure 3 shows the effects of several anti-Oncostatin M monoclonal antibodies on the binding of ¹²⁵I-Oncostatin M to the H2981 lung carcinoma cell line in the radioreceptor assay.

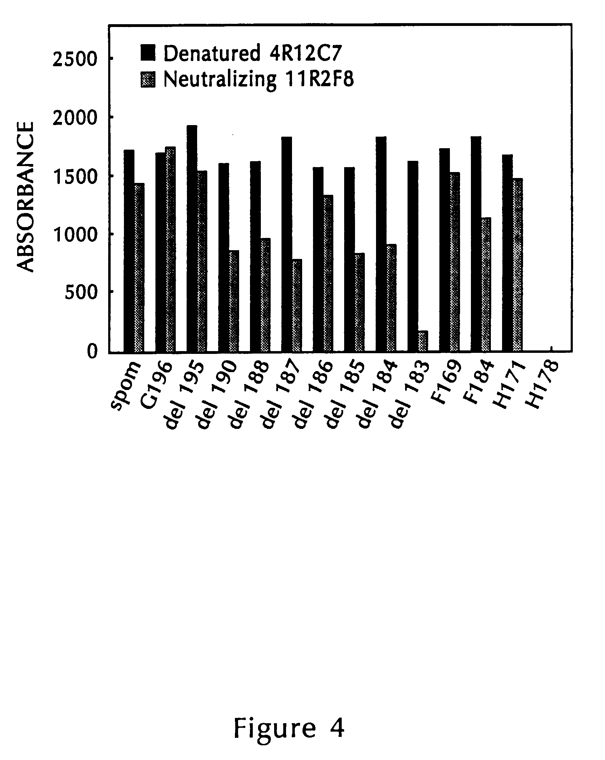

Figure 4 shows the binding, as detected by EIA, of two different anti-Oncostatin M monoclonal antibodies to supernatants secreted by COS cells transfected with a series of mutant Oncostatin M constructs containing amino acid deletions or alterations. Data are presented as total absorbance units at OD₄₆₀. Background binding is not subtracted. In this figure, "del" indicates the last amino acid which is deleted from the C-terminus, while alphabetical letters indicate the amino acid alteration made.

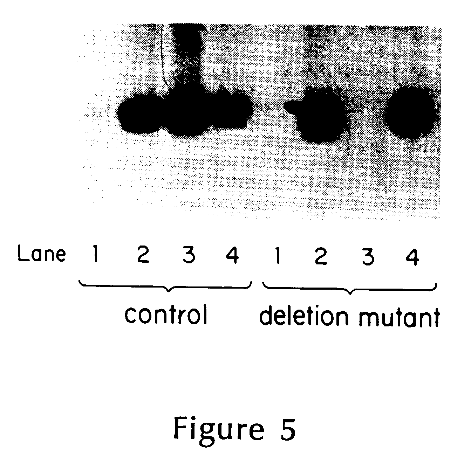

Figure 5 shows a comparison of the abilities of different anti-Oncostatin M monoclonal antibodies to immunoprecipitate Oncostatin M secreted by either the parental construct, "SPOM", or the deletion mutant △44-47. Lane 1: negative control antibody; lane 2: OM1; lane 3: OM2; lane 4: OM3.

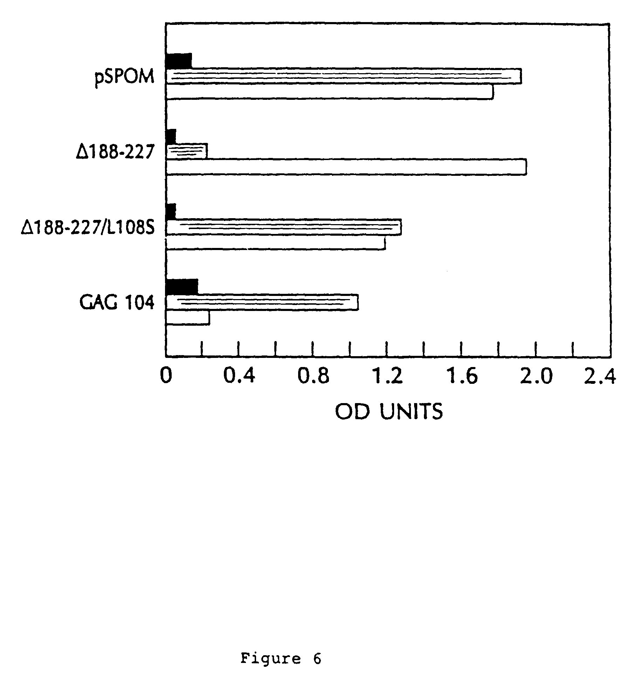

Figure 6 shows the mapping of the epitopes detected by two different anti-Oncostatin M monoclonal antibodies, OM3 and OM4. The relative binding, in absorbance units, of OM3 and OM4 is compared to that of a negative control antibody on OM from serum-free conditioned medium of COS cells transfected with plasmids △188-227 △188-227/L 108S, and GAG 104. Binding levels are compared to that of OM secreted from COS cells ("SPOM") (Linsley, et al., 1990, Mol. Cell. Biol. 10:1882-1890.)

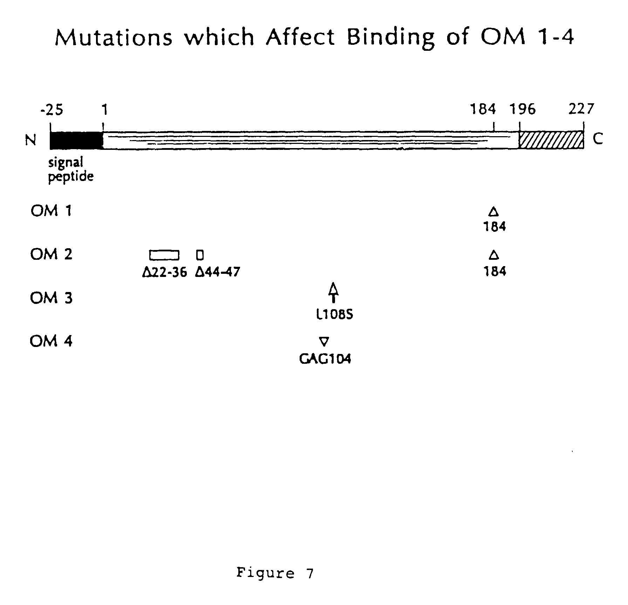

Figure 7 is a schematic diagram of mutations of Oncostatin M which affect the binding of a series of monoclonal antibodies directed against Oncostatin M. The leader sequence of OM is located from residues -25 to -1. The unprocessed molecule secreted from COS cells is 227 amino acids in length, and is cleaved to a mature 196 amino acid protein. (Linsley, et al., 1990, Mol. Cell. Biol. 10:1882-1890.) The deletion of C-terminal amino acids internal to and including 184 (△) destroys the binding of both OM1 and OM2. Additionally, OM2 binding is abrogated by deletions of residues 22-36 or 44-47. The epitope of antibody OM3 is mapped to a site containing residue 108 (arrow) since a change from leucine to serine at this residue destroys binding of this mAb. The insertion of the glycine-alanine-glycine tripeptide at residue 104 (∇) abolishes OM4 binding.

The present invention relates to monoclonal antibodies specific for Oncostatin M, a novel cytokine which exhibits pleiotropic effects on a wide variety of normal and transformed cells. Monoclonal antibodies which bind Oncostatin M, inhibit Oncostatin M receptor binding, and/or inhibit Oncostatin M bioactivity are described.

The monoclonal antibodies described can be used to map epitopes of Oncostatin M and to define structure-function relationships of its domains. Such antibodies may be used in diagnostic assays, for example, to detect the presence of Oncostatin M, or mutant forms of Oncostatin M. Alternatively, the monoclonal antibodies may be employed to modulate Oncostatin M biological activities in an in vivo or in vitro system. The invention is described in detail in the subsections below.

### CHARACTERISTICS OF MONOCLONAL ANTIBODIES DEFINED BY THEIR SPECIFICITY FOR ONCOSTATIN M

Monoclonal antibodies which define various epitopes of native and/or denatured forms of Oncostatin M are described. The monoclonal antibodies are further classified by their ability to block Oncostatin M biological activity and/or binding to cell surface receptors. Any monoclonal antibody, including chimeric antibodies, which competitively inhibit the immunospecific binding of the monoclonal antibodies described to their Oncostatin M epitopes are within the scope of the invention.

### ONCOSTATIN M ANTIGEN RECOGNITION

Oncostatin M, originally identified for its inhibitory effects on human tumor cell lines, was first isolated from phorbol 12-myristate 13-acetate ("PMA")-induced human histiocytic lymphoma cells (Zarling et al., 1986, Proc. Natl. Acad. Sci. (USA) 83: 9739-9743) and from activated T lymphocytes (Brown et al., 1987, J. Immunol. 139: 2977-2983). The molecule is a heat and acid stable protein comprised of a single polypeptide chain of Mᵣ = 28,000. Like other naturally occurring growth regulators, Oncostatin M exhibits a variety of biological activities. Growth inhibition is observed with some, but not all, human tumor cell lines. In contrast, the growth of some normal fibroblasts, such as human foreskin fibroblasts or WI-38 cells, is stimulated by exposure to Oncostatin M (Zarling et al., 1986, Proc. Natl. Acad. Sci. (USA) 83: 9739-9743). The gene for Oncostatin M has been cloned and sequenced, and an active form of recombinant Oncostatin M has recently been expressed in mammalian cells. (See U.S. Serial No. 144,574, filed January 15, 1988, equivalent to published European Patent Application EPA 0 290 948 (published November 17, 1988)). The mature form, after cleavage of the signal peptide, is a glycoprotein containing 227 amino acids, five of which are cysteine residues. The protein has an extremely hydrophilic carboxy terminal domain. Although Oncostatin M is not structurally related to other known cytokines, its mRNA contains an AU-rich region at its 3' untranslated end. This region in the Oncostatin M message is homologous to that of many cytokines, lymphokines and other growth-regulatory molecules, suggesting a common mode of regulating gene expression. A cellular receptor for Oncostatin M has been found on a variety of mammalian cells. The major Oncostatin M receptor molecule is a specific protein of Mᵣ = 150,000-160,000 (Linsley et al., 1989, J. Biol. Chem. 264: 4282-4289).

Oncostatin M may be obtained by techniques well known in the art from a variety of cell sources which synthesize bioactive Oncostatin M including, for example, cells which naturally produce Oncostatin M and cells transfected with recombinant DNA molecules capable of directing the synthesis and/or secretion of Oncostatin M. Alternatively, Oncostatin M may be synthesized by chemical synthetic methods including but not limited to solid phase peptide synthesis. Methods for the production of Oncostatin M are described in U.S. Serial No. 144,574, filed January 15, 1988, equivalent to published European Patent Application EPA 0 290 948 (published November 17, 1988).

Monoclonal antibodies with an affinity for Oncostatin M may be selected by assaying their capacity for binding Oncostatin M using any of a number of immunological assays, including but not limited to enzyme linked immunosorbant assay (ELISA), immunoprecipitation, Western blot analysis, radio-immunometric assays, competitive and non-competitive immunoassays. For example, the solid phase micro-enzyme assay ("MicroEIA") described below may be readily used. Briefly, antibodies found in the supernatant of hybrids are assessed for their ability to bind to Oncostatin M coated to a solid surface in wells. Following the addition of the supernatant, peroxidase-conjugated F(ab')₂ goat anti-mouse Ig is added to the well. After washing away any unbound materials, the bound enzyme is revealed by addition of a substrate which undergoes a color change. The color change indirectly indicates a monoclonal antibody/Oncostatin M complex formed in the well.

### INHIBITION OF ONCOSTATIN M ACTIVITY

Antibodies which inhibit the biological activity of Oncostatin M may find particular use in therapeutic applications. Such antibodies can be identified using the Growth Inhibition Assay ("GIA") as described below. Briefly, GIA provides a test system to assess the ability of an antibody to neutralize the inhibitory effects of Oncostatin M on the growth and proliferation of target cells.

### INHIBITION OF BINDING TO ONCOSTATIN M RECEPTOR

Cell surface receptors generally have a high affinity for their ligand. The binding of the ligand to the specific cell surface receptor initiates the control of various cellular events. Binding of Oncostatin M to a membrane receptor has been demonstrated using the radioreceptor assay described below and in U.S. Serial No. 144,574, filed January 15, 1988, referenced earlier. The human tumor cells tested included A375 (melanoma); A875 (melanoma); Me1109 (melanoma); T24 (bladder carcinoma); A549 (lung adenocarcinoma); H1477 (melanoma); Me180 (melanoma); and MCF (breast). Binding of ¹²⁵I-Oncostatin M was specific and saturable, and was not inhibited by other known polypeptide growth regulators. Scatchard analysis of binding data obtained with different cell lines revealed that ¹²⁵I-Oncostatin M bound to 1-2x10⁴ binding sites per cell with a K_{d} of approximately 10⁻⁹ M. The monoclonal antibodies produced by the hybrid cell lines were tested, using the radioreceptor assay described below, for their ability to block the binding of Oncostatin M to its cell surface receptor.

### METHODS FOR PREPARING MONOCLONAL ANTIBODIES TO ONCOSTATIN M

The anti-Oncostatin M antibodies of the invention can be prepared using any of a variety of techniques in which Oncostatin M is used as an immunogen which is injected into a mammalian host, e.g. mouse, cow, goat, sheep, rabbit, etc., particularly with an adjuvant, e.g. complete Freund's adjuvant, aluminum hydroxide gel, or the like. The host may then be bled and the blood employed for isolation of polyclonal antibodies. Alternatively, the peripheral blood lymphocytes, splenic lymphocytes (B-cells), or lymph node lymphocytes may be employed for fusion with an appropriate myeloma cell to immortalize the chromosomes for monoclonal expression of antibodies specific for Oncostatin M.

While the invention is described by way of examples using mouse monoclonal antibodies, the invention is not so limited and encompasses the use of, for example, human antibodies. Such antibodies can be obtained by using human hybridomas (Cote et al., 1983, Proc. Natl. Acad. Sci (U.S.A.), 80: 2026-2030) or by transforming human B cells with EBV (Epstein Barr Virus) in vitro (Cole et al.,1985, in "Monoclonal Antibodies and Cancer Therapy", Alan R. Liss, pp. 77-96). Techniques recently developed for the production of "chimeric antibodies" may be employed (Morrison et al., 1984, Proc. Natl. Acad. Sci. (U.S.A.), 81: 6851-6855; Neuberger et al., 1984, Nature 312: 604-608; Takeda et al., 1985, Nature 314: 452-454). These latter techniques involve splicing the genes from a mouse antibody molecule of appropriate antigen specificity together with genes from a human antibody molecule of appropriate biological activity.

A technique recently described can be used to generate a repertoire of monoclonal antibodies that define Oncostatin M (see Sastry, et al., 1989, Proc. Natl. Acad. Sci. (U.S.A.) 86: 5728-5732; and Huse et al., 1989, Science 246: 1275-1281). Accordingly, a cDNA library of Fab fragments derived from splenic DNA of animals primed with Oncostatin M can be generated in bacterial host cells.

The monoclonal antibodies of the invention can be modified by treatment with appropriate proteases, e.g., pepsin, papain, and the like, to generate Fab, F(ab')₂ or Fᵥ fragments that immunospecifically bind to Oncostatin M.

Additionally, the whole antibody molecule or its Fab, F(ab')₂ or Fᵥ fragment may be conjugated to any of a variety of compounds including, but not limited to, signal generating compounds such as a fluorescer, radiolabel, a chromophore, an enzyme, a chemoluminescent or bioluminescent molecule, etc. Alternatively, the whole antibody or its Fab, F(ab')₂ or Fᵥ fragment may be conjugated to a growth factor which may enhance or inhibit the biological activity of Oncostatin M; or to toxins so that cells which express Oncostatin M precursors on their surface would be selectively killed. Methods which can be used for conjugating labels, proteins, toxins, etc. to antibodies and antibody fragments are well known in the art. See, for example, U.S. Patent Nos. 4,220,450; 4,235,869; 3,935,074; and 3,996,345.

Thus, in an additional embodiment of the invention, there is provided a process for preparing a monoclonal antibody, or fragment thereof, the antigen combining site of which binds to Oncostatin M, which comprises
(a) culturing a cell line, said cell line capable of producing an anti-Oncostatin M antibody, or fragment thereof, and recovering the product produced; or,
(b) preparing an anti-Oncostatin M antibody fragment from an intact anti-Oncostatin M antibody; and, if desired,
(c) conjugating the antibody or antibody fragment prepared in (a) or (b) to a label, growth factor, or toxin.

### USES OF MONOCLONAL ANTIBODIES TO ONCOSTATIN M

The antibodies of the invention may be advantageously used to detect native or denatured forms of natural or recombinant Oncostatin M or to detect the presence of Oncostatin M in serum samples where it may occur in a free form or associated with its binding protein. Alternatively, the antibodies of the invention may be used in vivo to inhibit the biological effects of Oncostatin M. Either polyclonal or monoclonal antibodies may be used for the detection of Oncostatin M in a sample, such as cells or physiological fluid, e.g. blood. Detection of Oncostatin M in a body fluid may also be used as an indication of the presence of a tumor cell. In this regard, anti-Oncostatin M antibodies may be useful in the diagnosis and/or prognosis of cancer and/or other cell growth-related diseases. The antibodies may also be used in affinity chromatography for isolating and purifying Oncostatin M from natural or synthetic sources. The antibodies will also find use in controlling the amount of Oncostatin M associated with cells in culture or in vivo, whereby growth of the cells may be modified by the formation of a specific antibody:Oncostatin M complex resulting in competitive inhibition of Oncostatin M:Oncostatin M receptor binding. Thus, the antibodies of the invention may be useful as therapeutic agents in the treatment of cell growth disorders in which the growth stimulating activity of Oncostatin M is a factor.

Thus, for these purposes, there is provided a pharmaceutical formulation which comprises, as an active ingredient, an antibody of the invention, or fragment thereof, associated with a pharmaceutically acceptable diluent, excipient, or carrier therefor.

### GENERATION OF ANTI-IDIOTYPES THAT MIMIC THE EFFECTS OF ONCOSTATIN M

The monoclonal antibodies of the invention can also be used to generate anti-idiotypic antibodies that mimic the biological effects of Oncostatin M. Anti-idiotypic antibodies or anti-idiotypes are antibodies directed against the antigen-combining region or variable region (called the idiotype) of another antibody molecule. In theory, based on Jerne's network model of idiotypic relationships (Jerne, N.K., 1974, Ann. Immunol. (Paris) 125:373; Jerne, N.K. et al., 1982, EMBO 234), immunization with an antibody molecule expressing a paratope (antigen-combining site) for a given antigen should produce a group of anti-idiotypic antibodies, some of which share with the antigen a complementary structure to the paratope. Immunization with monoclonal antibodies that inhibit binding of Oncostatin M to its receptor should, in turn, produce anti-idiotypes that mimic Oncostatin M and bind to the Oncostatin M receptor. Thus it is within the scope of the invention that these anti-idiotypes can be produced by the monoclonal antibodies directed against Oncostatin M and which will mimic the effects of Oncostatin M in vivo and in vitro. Likewise, anti-idiotypic antibodies that bind to Oncostatin M are intended to be included in the definition of monoclonal antibodies that define Oncostatin M as used herein.

### ONCOSTATIN M EPITOPE MAPPING

Structural analysis of the growth regulator, Oncostatin M, is an important prelude to determining the biological role this novel cytokine plays in homeostasis or pathological states. In the examples described infra, a series of monoclonal antibodies (OM1 through OM8), produced against recombinant Oncostatin M, have been analyzed to determine their structural binding requirements and epitope localization. These antibodies detect either linear (OM3 and OM4) or folded epitopes (OM1 and OM2). The linear epitopes detected by OM3 and OM4 are situated in close proximity. OM3, whose epitope includes residue 108, reacts with both folded and denatured Oncostatin M, while Mab OM4, whose epitope is disrupted by insertion of a tripeptide at amino acid residue 104, binds only denatured Oncostatin M.

Monoclonal antibody OM2 abrogated the functional effects of Oncostatin M in a growth inhibition assay. The data presented infra indicate that antibody OM2 antagonizes the effects of OM by preventing OM from binding to its receptor. Through serological analysis, it has been determined that certain amino acid insertion, deletion, or substitution mutations affect the binding of the neutralizing antibody. The results of these experiments correlate very closely with those obtained by analysis of the GIA and RRA activity of these mutant molecules, and suggest that the neutralizing antibody binding site lies within the tertiary structure of the OM molecule which requires proper folding for biological activity.

The disruption of both receptor and OM2 binding in mutants having deletion or changes in non-contiguous (C-terminal, △22-36, △44-47) amino acid residues strengthens the interpretation that the functionally important region(s) of Oncostatin M is highly dependent on proper tertiary structure. These data additionally suggest that regions in both the N-terminus and C-terminus of the mature molecule are essential for the functional activity of Oncostatin M, whether directly by forming the receptor binding site or indirectly by stabilizing the tertiary structure necessary for activity.

Similar observations have been made for interleukin-6, which shares some functional properties with Oncostatin M including in vitro growth inhibition of certain tumor cell lines and induction of plasminogen activator inhibitor (Brown, et al., 1990, in "Molecular Biology of the Cardiovascular System, Elsevier, Amsterdam, pp. 195-206). Concomitant deletion of both the N-terminal amino acids past residue 31 and the first five carboxy terminal amino acids of Il-6 markedly reduces activity, while deletion of the sixteen C-terminal residues completely abolishes functional activity. A series of neutralizing antibodies to IL-6 appear to recognize epitopes formed by amino and carboxy terminal amino acids, and an alpha helical structure likewise has been proposed for the seven C-terminal amino acids of Il-6 (Brakenhoff, et al., 1990, J. Immunol. 145: 561-568. In contrast to the results presented for Oncostatin M, intramolecular disulfide bonds do not seem to be essential for Il-6 functional activity (Jambou, et al., 1988, Proc. Natl. Acad. Sci. (U.S.A.) 85: 9462-9430). Neutralizing monoclonal antibodies have been produced to α-interferon (Cebrian, et al., 1987, J. Immunol. 138: 484-490) and β-interferon (Redlich & Grossberg, 1989, J. Immunol. 143: 1887-1893) and, as with Oncostatin M, disulfide linkage is required for functionality. Epitope mapping on mutant interferon molecules has not been published. The compilation of structure-function analysis for these and other cytokines should aid in delineating how similar tertiary structural and functional features of these molecules can be accommodated by dissimilar primary structures. A schematic model of the functionally important regions of the Oncostatin M molecule determined so far with OM2, and with the mapping of the OM3 and OM4 epitopes, is presented in Figure 7.

The internal deletion mutant data reveal that, although the neutralizing antibody binding site is lost, the epitope detected by the non-neutralizing antibody, OM1, remains intact. Similar results have been obtained by alanine-scanning mutagenesis of the human growth hormone, in which numerous mutations can affect receptor binding without affecting binding of those monoclonal antibodies directed against epitopes other than the receptor binding region (Cunningham & Wells, 1989, Science 244: 1081-1085. These data indicate that local disruptions of the receptor binding site can occur without causing massive disruption of the tertiary structure of Oncostatin M. The mapping of the OM1 epitope has not been possible using the mutants generated so far. The OM1 epitope may depend on tertiary structure stabilized by the folding of the C-terminus.

Several complementary approaches to examining structure-function relationships of Oncostatin M have been described here and elsewhere. Because the antibody OM2 appears to exert its neutralizing activity by blocking Oncostatin M binding to it receptor, the potential exists for generating an anti-idiotypic antibody directed against the internal image of the neutralizing antibody which could recognize the Oncostatin M receptor. This approach has been applied successfully to the generation of antibodies directed against a number of different receptors, such as the insulin receptor (Sege & Peterson, 1978, Proc. Natl. Acad. Sci. (U.S.A.) 75: 2443-2444) and the β-adrenergic receptor (Schreiber, et al., 1980, Proc. Natl. Acad. Sci. (U.S.A.) 77: 7385-7389; Homey, et al., 1982, J. Clin. Invest. 69: 1147-1154), among others (Gaulton & Green, 1986, Ann. Rev. Immunol. 4: 253-280; Vaux, et al., 1990, Nature 345: 495-502). Localization of the epitopes detected by the monoclonal antibodies described here provides important tools with which the biological function and tissue distribution of Oncostatin M can be examined.

The following non-limiting Examples and Procedures are provided to further illustrate the invention.

### PRODUCTION OF MONOCLONAL ANTIBODIES TO ONCOSTATIN M

### MATERIALS AND METHODS

### A. IMMUNIZATION AND FUSION

The hybridomas were produced by immunization of 4 Balb/c mice with either 5 µg or 10 µg of recombinant Oncostatin M expressed by CHO cells transfected with the plasmid pBOM (Linsley, et al., 1989, J. Biol. Chem. 264: 4282-4289) and purified as described (Zarling, et al., 1986, Proc. Natl. Acad. Sci. (USA) 83: 9739-9743). The transfected CHO cells secrete Oncostatin M as a mature molecule of 196 amino acids which is processed from a 227 residue precursor molecule (Linsley, et al., 1990, Mol. Cell. Biol. 10: 1882-1890.

Briefly, the Oncostatin M was resuspended in PBS, emulsified in an equal volume of Complete Freund's Adjuvant, and 25 µg of the emulsion was injected into one hind footpad intradermally. Two weeks later, the same immunization protocol in the same hind footpad was followed, with concentrations of Oncostatin M identical to that for the first immunization. Two weeks after the second immunization, the mice were injected in the same hind footpad with a third preparation of Oncostatin M, this time emulsified in Incomplete Freund's Adjuvant. Three days after the final immunization, the popliteal lymph node was removed, and the lymph node cells were fused with murine myeloma 653/AG8 cells at a 1:1 lymph node cell to myeloma cell ratio, using 40% polyethylene glycol as a fusion agent. Fusion products at a concentration of 5 x 10⁴ cells/well were plated into 96-well plates containing hypoxanthine-aminopterin-thymidine as selection agent, in Iscoves Modification of Dulbecco's Modified Eagle's Medium ("IDMEM") supplemented with 10% fetal bovine serum, sodium pyruvate, and L-glutamine. After one week, original medium was replaced with fresh medium containing selection agent. Hybrids were screened by a solid-phase Micro Enzyme-linked Assay ("Micro EIA"), as described below, when the hybrids were visible macroscopically.

### B. ENZYME-LINKED IMMUNOASSAYS

The following Micro EIA was used to characterize the specificity of the antibodies to Oncostatin M produced by the hybridomas. Hybridoma supernatants were screened for activity using a solid-phase EIA adapted for use in Microtest plates (Robbins Scientific, San Francisco). Concentrations of purified Oncostatin M ranging from 2 to 4 µg/ml were plated in 5 µl volumes in each well, and allowed to air-dry overnight. After a one hour block with 5% nonfat dried milk in PBS and 0.1% sodium azide, the hybridoma supernatants were added in 1 µl volumes, and incubated at room temperature for 1 hour. The plates were washed six times by immersion in PBS. Peroxidase-conjugated F(ab')₂ goat anti-mouse IgG (Pel-Freez, Rogers AK) in PBS+2% BSA at a concentration of 1:1500 was added in 5 µl volumes to each well. After a 1 hour incubation, the plates were washed six times as previously described; 10 µl of substrate, 2,2'-azino-bis-(3-ethylbenzthiazoline-6-sulfonic acid) ("ABTS", Sigma Chemical Co., St. Louis, Mo.), was added, and plates were read at OD = 660 nm after 20 minutes. Positive wells were determined on the basis of signal above background relative to the signal of a previously generated rabbit antiserum against Oncostatin M. Positive hybrids were cloned in soft agar.

A double determinant EIA (DDEIA) was employed to evaluate the ability of monoclonal antibodies whose epitopes have tertiary structure to bind to a series of mutant Oncostatin M molecules. These mutants contained insertions, deletions, or substitutions in amino acid residues at various sites in the molecule. A direct EIA employing mAb OM3 was utilized to assess concentrations of the mutant molecules in the supernatants prior to their analysis in the DDEIA. For the DDEIA, 100 µl of Protein G (Pharmacia) affinity-purified monoclonal antibody OM3 at a concentration of 10 µg/ml in 0.05 M carbonate buffer, pH 9.6, was added to 96-well flat-bottomed plates, and incubated overnight at 4°C. The antibody was removed, and after one hour of blocking with PBS, 1% BSA, 0.05% Tween 20, either purified Oncostatin M at defined concentrations or serial dilutions of supernatants from COS cells transfected with plasmids encoding mutant forms of OM was added. The plates were incubated for 2 hours at 37°C, washed five times with PBS, and incubated again for 1 hour at 37°C with 100 µl of a 200 ng/ml concentration of biotinylated monoclonal antibody (OM1 or OM2). After a 1 hour incubation at 37°C, plates were washed 5 times; 100 µl of a 1:10,000 dilution of HRP-conjugated streptavidin (Vector) were added and the plates incubated for 30 minutes at 37°C. After washing, the reaction was developed with 3,3',5,5' tetramethylbenzidine ("TMB"), stopped with 1 N sulfuric acid, and A₄₅₀ was read.

### C. IMMUNOPRECIPITATION

CHO cells transfected with the Oncostatin-M-encoding cDNA (Linsley, et al., 1989, J. Biol. Chem. 264: 4282-4289) were incubated for 4-8 hours with 200 µCi each of ³⁵S-methionine and ³⁵S-cysteine in a volume of 4 ml in a 60 x 15 cm petri dish. Supernatants were collected and filtered; 10 mM phenylmethylsulfonyl fluoride ("PMSF") and 100 mM tosyl-lysine-chloromethylketone ("TLCK") were added to inhibit proteases. For immunoprecipitations, 100-200 µl of labelled supernatant were incubated overnight at 4°C with rotating with 200 µl of spent supernatant from the monoclonal antibody-secreting hybrids. Antigen-antibody complexes were isolated by incubation with a monoclonal rat anti-mouse κ light chain monoclonal antibody covalently coupled to Reactigel beads (Pierce Chemicals). After washing, the antigen-antibody complexes were eluted by boiling in sample buffer containing 1% SDS and 5% 2-mercaptoethanol. The eluted material was electrophoresed in 15% SDS-PAGE. Gels were fluorographed with Amplify™ (Amersham Corp., Arlington Heights, IL), dried, and autoradiographed with Kodak X-AR5 X-ray film.

### D. GROWTH INHIBITORY ASSAY

The antibodies were tested to determine whether any could neutralize the inhibitory effects of Oncostatin M in a Growth Inhibitory Assay ("GIA"). The GIA employs A375 melanoma cells (4x10³ cells/50 µl) as indicator cells. A375 cells are subcultured for four hours on flat-bottomed 96-well tissue culture plates (Costar 3595, Cambridge, MA) in growth medium comprising Dulbecco's modified Eagle's medium ("DMEM"), supplemented with 10% heat inactivated fetal bovine serum, and penicillin/streptomycin. Oncostatin M is diluted in growth medium and assayed for growth inhibition in triplicate by adding 50 µl of the diluted sample per well. The cells are then incubated for 72 hours at 37°C. At the end of this incubation period, each well is treated for 24 hours with 100 µl of growth medium containing [¹²⁵I]iodo-2'-deoxyuridine (0.05 µCi/well (Amersham Corp., Arlington Heights, IL)). The monolayers are washed with phosphate-buffered saline ("PBS"), fixed in 95% methanol and air-dried. The [¹²⁵I]-iododeoxyuridine incorporated by the cells is solubilised with 200 µl of 1 N sodium hydroxide and the amount of cell growth measured by the amount of [¹²⁵I]-iododeoxyuridine incorporated into the DNA of actively growing cells. One unit of activity is defined as the amount of the Oncostatin M required to give 50% inhibition of growth of A375 cells relative to untreated cells.

### E. ONCOSTATIN M RADIORECEPTOR ASSAY

Antibodies were tested in a radioreceptor assay for the ability to inhibit the binding of ¹²⁵I-labeled Oncostatin M to cell surface receptors for Oncostatin M on the human carcinoma cell line H2981. H2981 cells are plated in 48-well plates at a density of 2x10⁵ cells/well and maintained at 37°C for 16-24 hours before initiation of the assay. Cell monolayers are then washed once with Binding Buffer (Linsley et al., 1986, Biochemistry 25: 2978-2986). To measure total binding, ¹²⁵I-Oncostatin M was added at concentrations ranging from 0.5-100 ng/ml. To measure non-specific binding, unlabeled Oncostatin M is added simultaneously with the ¹²⁵I-Oncostatin M to replicate plates at a concentration 20 to 100-fold higher than the concentration of ¹²⁵I-Oncostatin M. Binding is allowed to proceed for 2-5 hours at 23°C, then the monolayers are washed four times with Binding Buffer. Cell-bound radioactivity is solubilized with 1 N NaOH and counted in a gamma counter. Specific binding is calculated by subtracting the non-specific binding from total binding. The dissociation constant (K_{d}) and binding capacity was determined by Scatchard analysis (Scatchard, 1949, Ann. N.Y. Acad. Sci. 51:660).

### F. ONCOSTATIN M MUTANTS

Anti-Oncostatin M monoclonal antibodies were tested against a series of recombinant Oncostatin M mutants, having insertions, deletions or substitutions, generated at the DNA level. The antibodies were tested as spent supernatants in an EIA on media from COS cells which were transfected transiently with the various parental and mutant constructs.

### G. DETECTION OF ONCOSTATIN M IN SERUM WITH MONOCLONAL ANTIBODIES

The presence of Oncostatin M in serum was detected as follows: Human serum samples were first applied to an S-300 sizing column to separate serum proteins by molecular weight. Fractions collected from the S-300 column were examined for GIA activity, both before and after acidification of the serum samples. Before acidification, most serum samples exhibited no GIA activity in the void volume fractions. However, serum fractions which were acidified, then reneutralized before adding to the GIA, exhibited activity in fractions of molecular weights greater than 200 kilodaltons, suggesting that the Oncostatin M in serum is generally associated with a binding factor of greater than 200kd, and is inactive in this state. The various serum fractions were examined by Western blotting. Reaction of the Oncostatin M specific monoclonal antibodies with proteins found in the high molecular weight fractions corresponding to the molecular weight of Oncostatin M confirmed the presence of Oncostatin M in these fractions. The Oncostatin M found in serum has been purified, and N-terminal amino acid sequencing indicates that it is identical to the native and recombinant Oncostatin M previously obtained.

### RESULTS

### H. SELECTION OF MONOCLONAL ANTIBODIES WITH SPECIFICITY FOR ONCOSTATIN M

Two series of fusions were performed, consisting of four fusions in each series, with one lymph node from each mouse used per fusion. From the first series, four hybrids were examined more extensively. From the second, more successful series of fusions (in terms of numbers of positive hybrids identified in the MicroEIA), at least 20 other anti-Oncostatin M monoclonal antibodies were identified. From this number, the following hybrids were selected for further study based on relative signal in the EIA and ability to immunoprecipitate either native or denatured metabolically labelled Oncostatin M: OM2, OM7, OM3, and OM8. Based on the immunoprecipitation data described below, the hybrids were grouped into one of three categories based on their reactivities with either native Oncostatin M, denatured Oncostatin M, or both (Table I). Five of these monoclonal antibodies were then tested to determine whether any of them could neutralize the inhibitory effects of Oncostatin M in the Growth Inhibitory Assay. These results are described below.

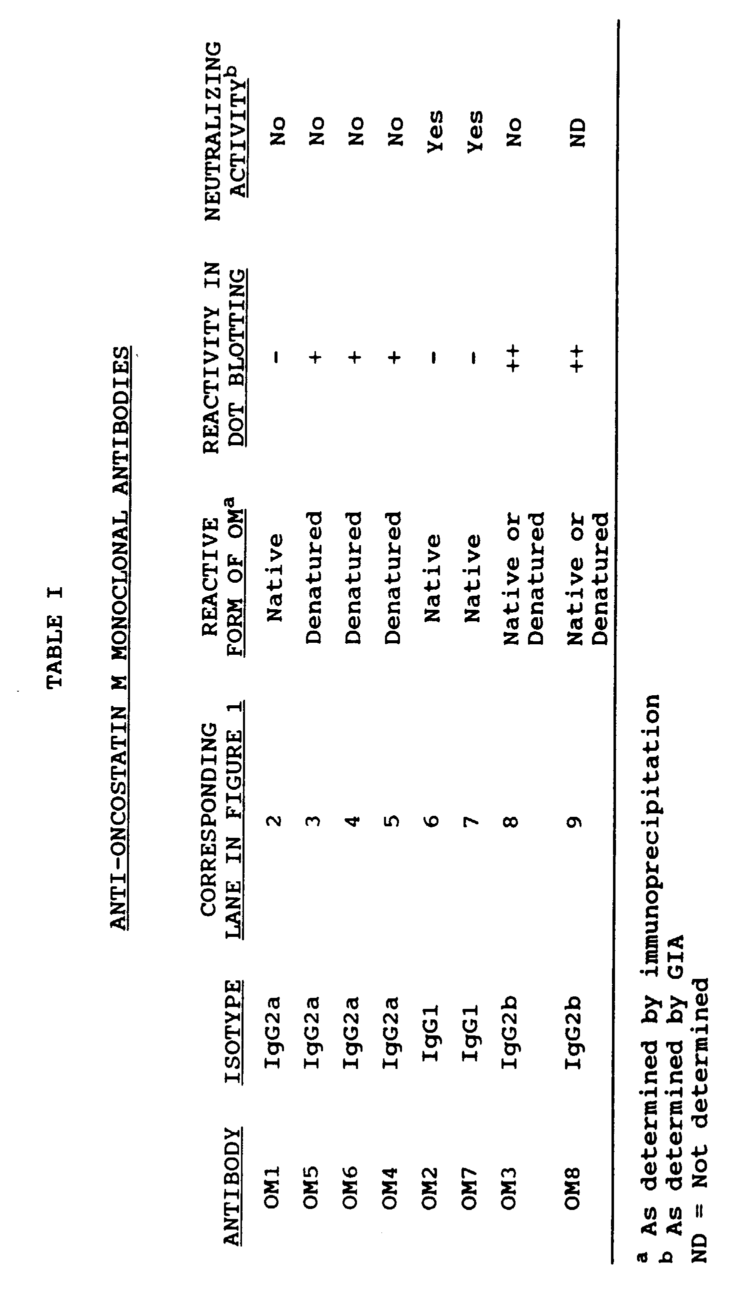

### I. MONOCLONAL ANTIBODIES IMMUNOPRECIPITATE WITH ONCOSTATIN M

Results of immunoprecipitation data identified three monoclonal antibodies (OM2, OM2 and OM7) reacting only with ³⁵S-methionine-labelled Oncostatin M in the form in which it was secreted from the CHO transfectant (group 1 (lanes 2, 6, 7; Figure 1; see Table I for identification of antibodies). Three additional monoclonal antibodies (OM4, OM5, and OM6) reacted only with ³⁵S-methionine-labelled Oncostatin M which was first reduced and denatured by treating with SDS and reducing agents, then boiled at 100°C, but not with native Oncostatin M (group 2, lanes 3, 4, 5). Two additional monoclonal antibodies (OM3 and OM8) reacted with biosynthetically labelled Oncostatin M whether it was native or denatured (group 3, lanes 8,9).

### J. MONOCLONAL ANTIBODIES THAT NEUTRALIZE ONCOSTATIN M IN THE GROWTH INHIBITION ASSAY

Purified antibody from the hybrid OM2 neutralized the effects of Oncostatin M in a concentration dependent fashion (Figure 2A). Antibody OM1, which is directed against native Oncostatin M, showed no blocking of growth inhibitory effects of Oncostatin M (Figure 2B). None of the antibodies detecting a denatured determinant was capable of neutralizing Oncostatin M as measured by GIA. The neutralizing antibody OM2 was further tested for its ability to inhibit Oncostatin M receptor binding, as described below.

### K. MONOCLONAL ANTIBODIES THAT INHIBIT BINDING OF ONCOSTATIN M IN THE RADIORECEPTOR ASSAY

The OM2 antibody was capable of inhibiting Oncostatin M receptor binding in a concentration dependent fashion, in contrast to either the OM1 antibody, directed against a native but non-neutralizing site, or a third antibody, OM4, directed against an epitope on denatured Oncostatin M (Figure 3).

### L. ANALYSIS OF FUNCTIONAL SITES AND EPITOPE MAPPING OF ONCOSTATIN M

The subsections below describe the characterization of monoclonal antibodies generated against recombinant OM purified from supernatants of transfected CHO cells (Linsley et al., 1989, J. Biol. Chem. 264: 4282-4289). Through serological analysis of products secreted by COS cells transfected with plasmids encoding a series of mutant forms of OM, we have mapped some of the epitopes detected by the monoclonal antibodies and determined some of the tertiary structural requirements for both antibody binding and functional activity of the OM molecule.

Of the two antibodies which detect folded epitopes, OM2, but not OM1, was identified as a neutralizing antibody based on its ability to abrogate OM activity in the growth inhibition assay (GIA) and to inhibit OM binding in the radioreceptor assay ("RRA"). Serological analysis of the mutant OM molecules demonstrated that the binding site of OM2 is affected by noncontiguous regions of OM, and that the presence of one of the two disulfide bonds (C₄₉-C₁₆₇) is essential for neutralizing antibody binding. In addition, certain mutations abrogate OM2 binding without causing global misfolding of the OM molecule.

These data indicate that the epitope defined by OM2 is spatially related to the binding site of OM, while those detected by OM1, OM3 and OM4 are distinct. The antibodies described here represent immunological probes for detecting OM in tissues and fluids of interest and will be useful in defining the physiological function and distribution of OM.

### M. MAPPING OF OM EPITOPES BY EIA ON ONCOSTATIN M MUTANTS

The growth regulator Oncostatin M (OM) is a novel cytokine which exhibits pleiotropic effects on a wide variety of normal and transformed cell lines. To determine some of the physiological functions of OM we have developed and characterized a series of monoclonal antibodies (OM1, OM2, OM3, and OM4) to the recombinant molecule as described in the subsections below.

Antibodies OM1 and OM2 bound native, but not denatured OM, suggesting they recognized epitopes with tertiary structural conformation. A third antibody, OM3, bound native or denatured OM, and antibody OM4 bound only denatured OM. Epitopes for these monoclonal antibodies (mAb) were localized by measuring antibody binding to a panel of mutant forms of OM. The OM3 binding site contains residue 108, while that for OM4 is disrupted by amino acid insertion at position 104.

In order to map the epitopes detected by these antibodies, we tested their binding to OM in serum-free conditioned medium from COS cells transfected with a series of plasmids encoding OM mutations in amino acid residues at various sites in the molecule. Antibodies OM3 and OM4, which detect linear epitopes, were examined in direct EIA for binding to a series of mutant OM proteins from which C-terminal amino acids were deleted, in sequential fashion, by means of stop codon insertions (Figure 6). Mutant △188-227 was bound by OM3, while △188-227/L108S, with an additional change from leucine to serine at residue 108, was not bound. Antibody OM4 was mapped to a site which was disrupted by the insertion of a glycine-alanine-glycine sequence at position 104 (Figure 6). Antibodies OM5 and OM6, which also reacted predominantly with denatured OM, did not react with the same epitope as OM4, since they reacted with the GAG104 mutant, which is not bound by OM4. None of the series of OM mutants generated thus far was informative in the epitope mapping of these mutants. Antibody OM7 is probably identical to antibody OM2, the neutralizing antibody, since it has the same Ig isotype and OM mutant binding patterns as OM2, while antibodies OM8 and OM3 are probably identical for the same reason.

### N. SEROLOGICAL ANALYSIS OF OM1 AND OM2 EPITOPES

In order to analyze the OM structural requirements for binding of antibody OM1 and OM2, both of which reacted only with folded forms of OM, we developed a double determinant EIA. The assay employed antibody OM3, which bound either native or denatured OM, to capture the OM from the COS transfectant mutant supernatants. The concentrations of OM (from the mutant molecules) bound by either biotinylated OM1 or OM2 were compared to a standard curve of purified native OM bound by the respective antibodies.

Results are presented in Table II as the concentration of OM mutant molecules bound by these antibodies, in ng/ml, compared to their binding of purified OM. Because this assay was saturated at the concentrations of OM present in undiluted supernatants of the mutant transfectants, serial dilutions were required to detect OM in the linear portion of the DDEIA. In all cases where the extrapolated values of detected OM were greater than 200 ng/ml, the absorbance values of the dilution series for the different mutant on molecules had the same slope, indicating that the biotinylated antibodies bound such mutant molecules with the same relative affinity.

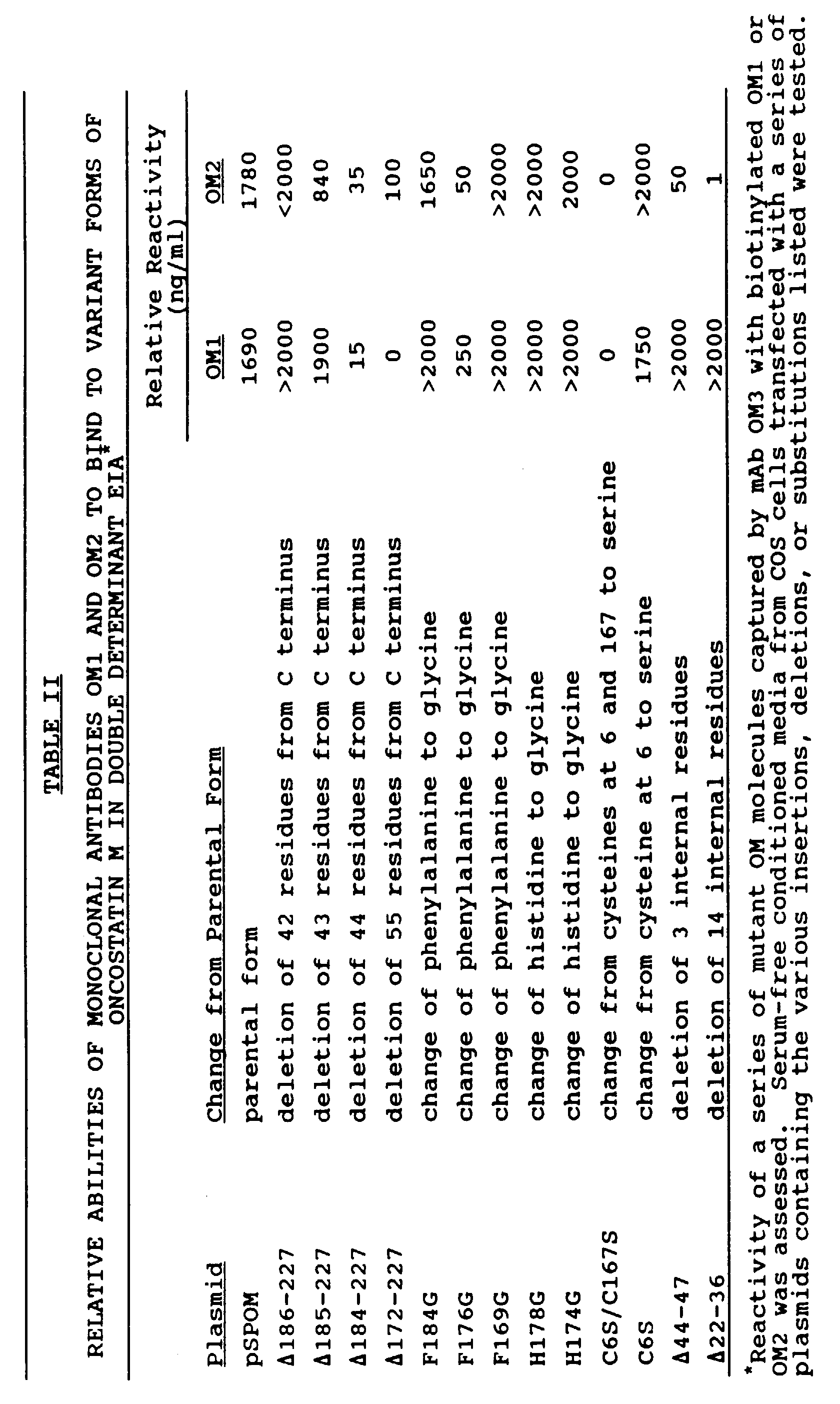

Structural analysis of the OM molecule indicated the presence of a strongly amphipathic/amphiphilic region near the C-terminus from amino acids 168-196. The OM1 and OM2 antibodies were tested for their binding to a series of mutants which had sequential C-terminal deletions. Both OM1 and OM2 were capable of detecting OM, with high affinity, from mutants which had sequential C terminal amino acid deletions up to amino acid 186 (as indicated by the high concentration of protein bound). For the △185-227 C-terminal deletion mutant, both antibodies bound with lower affinity; the binding level of OM2, the neutralizing antibody, was lower than that of OM1. Binding activity of both antibodies was completely lost in the C-terminal deletion mutant △184-227. A series of mutants of the amphiphilic region with residue changes from either hydrophobic or hydrophilic to neutral amino acids presented a more complex pattern of antibody binding. Of the three phenylalanine to glycine changes, only that at position 176 resulted in a complete loss of binding by both antibodies, while the change at 184 reduced the binding of the neutralizing antibody OM2, but not OM1. The substitution from phenylalanine to glycine at residue 169 had no effect on the binding of either antibody. Two separate proteins, H174G and H1786, with alterations of hydrophilic hisitidines to glycine at positions 174 and 178, respectively, were bound with high affinity by both OM1 and OM2.

Two intramolecular disulfide bonds exist in the native Oncostatin M molecule. The C₆-C₁₆₇ disulfide bond did not affect the local tertiary structure(s) of the OM1 and OM2 epitopes, since antibody binding was not reduced when the cysteine at position 6 was changed to serine. Elimination of both disulfide bonds (C6S/C167S) destroyed the epitopes of both antibodies, presumably by causing global misfolding of the molecule.

Two mutant OM molecules, Δ44-47, and Δ22-36, were informative in discriminating the binding requirements of antibodies OM1 and OM2. These deletion mutants were bound by OM1, the non-neutralizing antibody, but not by OM2, the neutralizing antibody. A schematic model of the functionally important regions of the Oncostatin M molecule determined with OM2, and with the mapping of OM3 and OM4 epitopes, is presented in Figure 7.

### M. DEPOSIT OF MICROORGANISMS

The following cell lines, representative of the present invention, have been deposited with the American Type Culture Collection, Rockville, Maryland, and have been assigned the listed accession numbers:

| Cell Line | Accession Number | Antibody |
|---|---|---|
| 11R2F8.9 | ATCC HB 10398 | OM2 |
| 12R13D7.2 | ATCC HB 10396 | OM3 |
| 1R10F11.34.16 | ATCC HB 10397 | OM1 |

The deposited cell lines are intended as single illustrations of individual aspects of the invention, and any cell line or antibody which is functionally equivalent is within the scope of this invention.

## Claims (Claims for the following Contracting State(s): AT, BE, CH, DE, DK, FR, GB, IT, LI, LU, NL, SE)

1. A monoclonal antibody, or fragment thereof, the antigen combining site of which binds to Oncostatin M and competitively inhibits the immunospecific binding of monoclonal antibody OM2, OM3, or OM1 produced by hybridoma cell lines 11R2F8 (ATCC HB 10398), 12R13D7 (ATCC HB 10396) and 1R10F11 (ATCC HB 10397), respectively, to its target antigen.

2. Monoclonal antibody OM2, OM3, or OM1 produced by hybridoma cell lines 11R2F8 (ATCC HB 10398), 12R13D7 (ATCC HB 10396) and 1R10F11 (ATCC HB 10397), respectively, or a fragment thereof.

3. An Fab, F(ab')₂, or Fv fragment of a monoclonal antibody as claimed in any one of Claims 1 and 2.

4. A monoclonal antibody, or fragment thereof, as claimed in any one of Claims 1 to 3, conjugated to a label capable of producing a detectable signal.

5. A monoclonal antibody, or fragment thereof, as claimed in Claim 4, in which the label comprises a fluorescer, a radiolabel, a chromophore, or an enzyme.

6. A monoclonal antibody, or fragment thereof, as claimed in any one of Claims 1 to 3, which is conjugated to a growth factor or toxin.

7. A cell line capable of producing a monoclonal antibody, or antibody fragment thereof, as claimed in any one of Claims 1 to 3.

8. Hybridoma cell line 11R2F8 (ATCC HB 10398), 12R13D7 (ATCC HB 10396), or 1R10F11 (ATCC HB 10397).

9. A monoclonal antibody, or fragment thereof, as defined in any one of Claims 1 to 6, for use in the diagnosis or treatment of a disease state which involves the biological activity of Oncostatin M.

10. A method for the detection of Oncostatin M which comprises contacting a biological sample with an antibody, or fragment thereof, as defined in any one of Claims 1 to 6.

11. A process for preparing a monoclonal antibody as defined in any one of Claims 1 to 6, or fragment thereof, the antigen combining site of which binds to Oncostatin M, which comprises
(a) culturing a cell line, said cell line capable of producing an anti-Oncostatin M antibody, or fragment thereof, and recovering the product produced; or,
(b) preparing an anti-Oncostatin M antibody fragment from an intact anti-Oncostatin M antibody; and, if desired,
(c) conjugating the antibody or antibody fragment prepared in (a) or (b) to a label, growth factor, or toxin.

12. A pharmaceutical formulation which comprises as active ingredient, at least one antibody, or fragment thereof, as claimed in any one of Claims 1 to 6, associated with a pharmaceutically acceptable carrier, diluent or excipient therefor.

13. The monoclonal antibody of any one of Claims 1 to 6, which is a chimeric antibody or a fragment thereof.

14. A cDNA molecule encoding the chimeric antibody or fragment of Claim 13.

## Claims (Claims for the following Contracting State(s): ES)

1. A process for preparing a monoclonal antibody, or fragment thereof, the antigen combining site of which binds to Oncostatin M and competitively inhibits the immunospecific binding of monoclonal antibody OM2, OM3, or OM1 produced by hybridoma cell lines 11R2F8 (ATCC HB 10398), 12R13D7 (ATCC HB 10396) and 1R10F11 (ATCC HB 10397), respectively, to its target antigen, which comprises
(a) culturing a cell line, said cell line capable of producing an anti-Oncostatin M antibody, or fragment thereof, and recovering the product produced; or,
(b) preparing an anti-Oncostatin M antibody fragment from an intact anti-Oncostatin M antibody; and, if desired,
(c) conjugating the antibody or antibody fragment prepared in (a) or (b) to a label, growth factor, or toxin.

2. A process as claimed in Claim 1 which produces monoclonal antibody OM2, OM3, or OM1 produced by hybridoma cell lines 11R2F8 (ATCC HB 10398), 12R13D7 (ATCC HB 10396) and 1R10F11 (ATCC HB 10397), respectively, or a fragment thereof.

3. A process as claimed in any one of Claims 1 or 2 in which an Fab, F(ab')₂, or Fv fragment of a monoclonal antibody is produced.

4. A process as claimed in any one of Claims 1 to 3 in which a monoclonal antibody, or fragment thereof, is conjugated to a label capable of producing a detectable signal.

5. A process as claimed in Claim 4 in which the label comprises a fluorescer, a radiolabel, a chromophore, or an enzyme.

6. A process as claimed in any one of Claims 1 to 3 in which the monoclonal antibody, or fragment thereof, is conjugated to a growth factor or toxin.

7. A cell line capable of producing the monoclonal antibody, or antibody fragment thereof, as defined in any one of Claims 1 to 3.

8. A process for producing the hybridoma cell lines 11R2F8 (ATCC HB 10398), 12R13D7 (ATCC HB 10396), or 1R10F11 (ATCC HB 10397), which comprises fusing lymphocytes of an Oncostatin M immunized mammalian host with an appropriate myeloma cell and selecting the hybridoma cell.

9. A process for preparing a pharmaceutical formulation which comprises admixing a monoclonal antibody, or fragment thereof, as defined in any one of Claims 1 to 6, with a pharmaceutically acceptable carrier, diluent or excipient.

10. The process of Claim 1 producing a chimeric antibody or a fragment thereof.

11. A process of preparing a cDNA molecule, which process comprises providing a cDNA sequence encoding the chimeric antibody or fragment thereof as defined in Claim 10.

## Claims (Claims for the following Contracting State(s): GR)

1. A monoclonal antibody, or fragment thereof, the antigen combining site of which binds to Oncostatin M and competitively inhibits the immunospecific binding of monoclonal antibody OM2, OM3, or OM1 produced by hybridoma cell lines 11R2F8 (ATCC HB 10398), 12R13D7 (ATCC HB 10396) and 1R10F11 (ATCC HB 10397), respectively, to its target antigen.

2. Monoclonal antibody OM2, OM3, or, OM1 produced by hybridoma cell lines 11R2F8 (ATCC HB 10398), 12R13D7 (ATCC HB 10396) and 1R10F11 (ATCC HB 10397), respectively, or a fragment thereof.

3. An Fab, F(ab')₂, or Fv fragment of a monoclonal antibody as claimed in any one of Claims 1 and 2.

4. A monoclonal antibody, or fragment thereof, as claimed in any one of Claims 1 to 3, conjugated to a label capable of producing a detectable signal.

5. A monoclonal antibody, or fragment thereof, as claimed in Claim 4, in which the label comprises a fluorescer, a radiolabel, a chromophore, or an enzyme.

6. A monoclonal antibody, or fragment thereof, as claimed in any one of Claims 1 to 3, which is conjugated to a growth factor or toxin.

7. A cell line capable of producing a monoclonal antibody, or antibody fragment thereof, as claimed in any one of Claims 1 to 3.

8. Hybridoma cell line 11R2F8 (ATCC HB 10398), 12R13D7 (ATCC HB 10396), or 1R10F11 (ATCC HB 10397).

9. A monoclonal antibody, or fragment thereof, as defined in any one of Claims 1 to 6, for use in the diagnosis or treatment of a disease state which involves the biological activity of Oncostatin M.

10. A method for the detection of Oncostatin M which comprises contacting a biological sample with an antibody, or fragment thereof, as defined in any one of Claims 1 to 6.

11. A process for preparing a monoclonal antibody as defined in any one of Claims 1 to 6, or fragment thereof, the antigen combining site of which binds to Oncostatin M, which comprises
(a) culturing a cell line, said cell line capable of producing an anti-Oncostatin M antibody, or fragment thereof, and recovering the product produced; or,
(b) preparing an anti-Oncostatin M antibody fragment from an intact anti-Oncostatin M antibody; and, if desired,
(c) conjugating the antibody or antibody fragment prepared in (a) or (b) to a label, growth factor, or toxin.

12. A process for preparing a pharmaceutical formulation which comprises admixing a monoclonal antibody, or fragment thereof, as claimed in any one of Claims 1 to 6, with a pharmaceutically acceptable carrier, diluent or excipient.

13. The monoclonal antibody of any one of Claims 1 to 6, which is a chimeric antibody or a fragment thereof.

14. A cDNA molecule encoding the chimeric antibody or fragment of Claim 13.

## Patentansprüche (Patentansprüche für folgende(n) Vertragsstaat(en): AT, BE, CH, DE, DK, FR, GB, IT, LI, LU, NL, SE)

1. Monoklonaler Antikörper oder ein Fragment davon, dessen Antigenbindungsstelle an Oncostatin M bindet und die immunspezifische Bindung der von den Hybridomzellinien 11R2F8 (ATCC HB 10398), 12R13D7 (ATCC HB 10396) bzw. 1R10F11 (ATCC HB 10397) produzierten Antikörper OM2, OM3 oder OM1 an das Target-Antigen kompetitiv inhibiert.

2. Monoklonaler Antikörper OM2, OM3 oder OM1, produziert von den Hybridomzellinien 11R2F8 (ATCC HB 10398), 12R13D7 (ATCC HB 10396) bzw. 1R10F11 (ATCC HB 10397), oder ein Fragment davon.

3. Fab-, F(ab')₂- oder Fv-Fragment eines monoklonalen Antikörpers nach einem der Ansprüche 1 und 2.

4. Monoklonaler Antikörper oder ein Fragment davon nach einem der Ansprüche 1 bis 3, gebunden an eine Markierung, die in der Lage ist, ein detektierbares Signal zu erzeugen.

5. Monoklonaler Antikörper oder ein Fragment davon nach Anspruch 4, wobei es sich bei der Markierung um eine fluoreszierende Substanz, eine radioaktive Markierung, ein Chromophor oder ein Enzym handelt.

6. Monoklonaler Antikörper oder ein Fragment davon nach einem der Ansprüche 1 bis 3, gebunden an einen Wachstumsfaktor oder ein Toxin.

7. Zellinie, die in der Lage ist, einen monoklonalen Antikörper oder ein Antikörperfragment davon nach einem der Ansprüche 1 bis 3 zu produzieren.

8. Hybridomzellinie 11R2F8 (ATCC HB 10398), 12R13D7 (ATCC HB 10396) oder 1R10F11 (ATCC HB 10397).

9. Monoklonaler Antikörper oder ein Fragment davon nach einem der Ansprüche 1 bis 6 zur Verwendung für die Diagnose oder Behandlung eines Krankheitszustandes, an dem die biologische Aktivität von Oncostatin M beteiligt ist.

10. Verfahren zur Detektion von Oncostatin M, wobei man eine biologische Probe mit einem Antikörper oder einem Fragment davon nach einem der Ansprüche 1 bis 6 in Kontakt bringt.

11. Verfahren zur Herstellung eines monoklonalen Antikörpers nach einem der Ansprüche 1 bis 6 oder eines Fragmentes davon, dessen Antigenbindungsstelle an Oncostatin M bindet, umfassend die
(a) Kultivierung einer Zellinie, die in der Lage ist einen Anti-Oncostatin M-Antikörper oder ein Fragment davon zu produzieren, und Isolierung des erhaltenen Produktes; oder
(b) Herstellung eines Anti-Oncostatin M-Antikörperfragmentes aus einem intakten Anti-Oncostatin M-Antikörper; und gegebenenfalls
(c) Bindung des in (a) oder (b) hergestellten Antikörpers oder Antikörperfragmentes an eine Markierung, einen Wachstumsfaktor oder ein Toxin.

12. Pharmazeutische Formulierung, umfassend als Wirkstoff wenigstens einen Antikörper oder ein Fragment davon nach einem der Ansprüche 1 bis 6, zusammen mit einem pharmazeutisch verträglichen Träger, Verdünnungsmittel oder Exzipienten dafür.

13. Monoklonaler Antikörper nach einem der Ansprüche 1 bis 6, wobei es sich um einen chimären Antikörper oder ein Fragment davon handelt.

14. cDNA-Molekül, welches für den chimären Antikörper oder dessen Fragment gemäß Anspruch 13 kodiert.

## Patentansprüche (Patentansprüche für folgende(n) Vertragsstaat(en): ES)

1. Verfahren zur Herstellung eines monoklonalen Antikörpers oder eines Fragmentes davon, dessen Antigenbindungsstelle an Oncostatin M bindet und die immunspezifische Bindung der von den Hybridomzellinien 11R2F8 (ATCC HB 10398), 12R13D7 (ATCC HB 10396) bzw. 1R10F11 (ATCC HB 10397) produzierten Antikörper OM2, OM3 oder OM1 an das Target-Antigen kompetitiv inhibiert, umfassend die
(a) Kultivierung einer Zellinie, die in der Lage ist einen Anti-Oncostatin M-Antikörper oder ein Fragment davon zu produzieren, und Isolierung des erhaltenen Produktes; oder
(b) Herstellung eines Anti-Oncostatin M-Antikörperfragmentes aus einem intakten Anti-Oncostatin M-Antikörper; und gegebenenfalls
(c) Bindung des in (a) oder (b) hergestellten Antikörpers oder Antikörperfragmentes an eine Markierung, einen Wachstumsfaktor oder ein Toxin.

2. Verfahren nach Anspruch 1 zur Herstellung des monoklonalen Antikörpers OM2, OM3 oder OM1, produziert von den Hybridomzellinien 11R2F8 (ATCC HB 10398), 12R13D7 (ATCC HB 10396) bzw. 1R10F11 (ATCC HB 10397), oder eines Fragmentes davon.

3. Verfahren nach einem der Ansprüche 1 oder 2, wobei ein Fab-, F(ab')₂- oder Fv-Fragment eines monoklonalen Antikörpers produziert wird.

4. Verfahren nach einem der Ansprüche 1 bis 3, wobei ein monoklonaler Antikörper oder ein Fragment davon an eine Markierung gebunden ist, die in der Lage ist, ein detektierbares Signal zu erzeugen.

5. Verfahren nach Anspruch 4, wobei es sich bei der Markierung um eine fluoreszierende Substanz, eine radioaktive Markierung, ein Chromophor oder ein Enzym handelt.

6. Verfahren nach einem der Ansprüche 1 bis 3, wobei der monoklonale Antikörper oder dessen Fragment an einen Wachstumsfaktor oder ein Toxin gebunden ist.

7. Zellinie, die in der Lage ist, einen monoklonalen Antikörper oder ein Antikörperfragment davon nach einem der Ansprüche 1 bis 3 zu produzieren.

8. Verfahren zur Herstellung der Hybridomzellinien 11R2F8 (ATCC HB 10398), 12R13D7 (ATCC HB 10396) oder 1R10F11 (ATCC HB 10397), wobei man die Lymphocyten eines gegen Oncostatin M immunisierten Wirtssäugetiers mit einer geeigneten Myelomzelle fusioniert und die Hybridomzelle selektiert.

9. Verfahren zur Herstellung einer pharmazeutischen Formulierung, wobei man einen monoklonalen Antikörper oder ein Fragment davon nach einem der Ansprüche 1 bis 6 mit einem pharmazeutisch verträglichen Träger, Verdünnungsmittel oder Exzipienten vermischt.

10. Verfahren nach Anspruch 1 zur Herstellung eines chimären Antikörpers oder eines Fragmentes davon.

11. Verfahren zur Herstellung eines cDNA-Moleküls, umfassend die Bereitstellung einer cDNA-Sequenz, die für den chimären Antikörper oder dessen Fragment gemäß Anspruch 10 kodiert.

## Patentansprüche (Patentansprüche für folgende(n) Vertragsstaat(en): GR)

1. Monoklonaler Antikörper oder ein Fragment davon, dessen Antigenbindungsstelle an Oncostatin M bindet und die immunspezifische Bindung der von den Hybridomzellinien 11R2F8 (ATCC HB 10398), 12R13D7 (ATCC HB 10396) bzw. 1R10F11 (ATCC HB 10397) produzierten Antikörper OM2, OM3 oder OM1 an das Target-Antigen kompetitiv inhibiert.

2. Monoklonaler Antikörper OM2, OM3 oder OM1, produziert von den Hybridomzellinien 11R2F8 (ATCC HB 10398), 12R13D7 (ATCC HB 10396) bzw. 1R10F11 (ATCC HB 10397), oder ein Fragment davon.

3. Fab-, F(ab')₂- oder Fv-Fragment eines monoklonalen Antikörpers nach einem der Ansprüche 1 und 2.

4. Monoklonaler Antikörper oder ein Fragment davon nach einem der Ansprüche 1 bis 3, gebunden an eine Markierung, die in der Lage ist, ein detektierbares Signal zu erzeugen.

5. Monoklonaler Antikörper oder ein Fragment davon nach Anspruch 4, wobei es sich bei der Markierung um eine fluoreszierende Substanz, eine radioaktive Markierung, ein Chromophor oder ein Enzym handelt.

6. Monoklonaler Antikörper oder ein Fragment davon nach einem der Ansprüche 1 bis 3, gebunden an einen Wachstumsfaktor oder ein Toxin.

7. Zellinie, die in der Lage ist, einen monoklonalen Antikörper oder ein Antikörperfragment davon nach einem der Ansprüche 1 bis 3 zu produzieren.

8. Hybridomzellinie 11R2F8 (ATCC HB 10398), 12R13D7 (ATCC HB 10396) oder 1R10F11 (ATCC HB 10397).

9. Monoklonaler Antikörper oder ein Fragment davon nach einem der Ansprüche 1 bis 6 zur Verwendung für die Diagnose oder Behandlung eines Krankheitszustandes, an dem die biologische Aktivität von Oncostatin M beteiligt ist.

10. Verfahren zur Detektion von Oncostatin M, wobei man eine biologische Probe mit einem Antikörper oder einem Fragment davon nach einem der Ansprüche 1 bis 6 in Kontakt bringt.

11. Verfahren zur Herstellung eines monoklonalen Antikörpers nach einem der Ansprüche 1 bis 6 oder eines Fragmentes davon, dessen Antigenbindungsstelle an Oncostatin M bindet, umfassend die
(a) Kultivierung einer Zellinie, die in der Lage ist einen Anti-Oncostatin M-Antikörper oder ein Fragment davon zu produzieren, und Isolierung des erhaltenen Produktes; oder
(b) Herstellung eines Anti-Oncostatin M-Antikörperfragmentes aus einem intakten Anti-Oncostatin M-Antikörper; und gegebenenfalls
(c) Bindung des in (a) oder (b) hergestellten Antikörpers oder Antikörperfragmentes an eine Markierung, einen Wachstumsfaktor oder ein Toxin.

12. Verfahren zur Herstellung einer pharmazeutischen Formulierung, wobei man einen monoklonalen Antikörper oder ein Fragment davon nach einem der Ansprüche 1 bis 6 mit einem pharmazeutisch verträglichen Träger, Verdünnungsmittel oder Exzipienten vermischt.

13. Monoklonaler Antikörper nach einem der Ansprüche 1 bis 6, wobei es sich um einen chimären Antikörper oder ein Fragment davon handelt.

14. cDNA-Molekül, welches für den chimären Antikörper oder dessen Fragment gemäß Anspruch 13 kodiert.

## Revendications (Revendications pour l'(les) Etat(s) contractant(s) suivant(s): AT, BE, CH, DE, DK, FR, GB, IT, LI, LU, NL, SE)

1. Anticorps monoclonal, ou fragment de celui-ci, dont le site de combinaison d'antigène se lie à l'Oncostatine M et inhibe compétitivement la liaison imunospécifique de l'anticorps monoclonal OM2, OM3, ou OM1 produit par les lignes de cellules d'hybridomes 11R2F8 (ATCC HB 10398), 12R13D7 (ATCC HB 10396) et 1R10F11 (ATCC HB 10397), respectivement, à son antigène cible.

2. Anticorps monoclonal OM2, OM3, ou OM1 produit par les lignes de cellules d'hybridomes 11R2F8 (ATCC HB 10398), 12R13D7 (ATCC HB 10396) et 1R10F11 (ATCC HB 10397), respectivement, ou fragment de celui-ci.

3. Fragment Fab, F(ab')₂ ou Fv d'un anticorps monoclonal selon l'une quelconque des revendications 1 et 2.

4. Anticorps monoclonal, ou fragment de celui-ci, selon l'une quelconque des revendications 1 à 3, conjugué à un marqueur capable de produire un signal détectable.

5. Anticorps monoclonal, ou fragment de celui-ci, selon la revendication 4, dans lequel le marqueur comprend un fluoresceur, un radio-marqueur, un chromophore, ou un enzyme.

6. Anticorps monoclonal, ou fragment de celui-ci, selon l'une quelconque des revendications 1 à 3, qui est conjugué avec un facteur de croissance ou une toxine.

7. Ligne de cellules capable de produire un anticorps monoclonal, ou un fragment d'anticorps de celui-ci, selon l'une quelconque des revendications 1 à 3.

8. Ligne de cellules d'hybridomes 11R2F8 (ATCC HB 10398), 12R13D7 (ATCC HB 10396), ou 1R10F11 (ATCC HB 10397).

9. Anticorps monoclonal, ou fragment de celui-ci, selon l'une quelconque des revendications 1 à 6, pour utilisation dans le diagnostic ou le traitement d'un état de maladie qui implique l'activité biologique de l'Oncostatine M.

10. Méthode de détection d'oncostatine. M qui comprend la mise en contact d'un échantillon biologique avec un anticorps, ou un fragment de celui-ci, tel que défini dans l'une quelconque des revendications 1 à 6.

11. Procédé pour préparer un anticorps monoclonal tel que défini dans l'une des revendications 1 à 6, où un fragment de celui-ci, dont le site de combinaison d'antigène se lie à l'Oncostatine M, qui comprend :
(a) la culture d'une ligne de cellules, ladite ligne de cellules capable de produire un anticorps anti-Oncostatine M, ou un fragment de celui-ci et la récupération du produit produit ; ou
(b) la préparation d'un fragment d'anticorps anti-Oncostatine M à partir d'un anticorps anti- Oncostatine M intact ; et, si souhaité,
(c) la conjugaison de l'anticorps ou du fragment d'anticorps préparé en (a) ou (b) à un marqueur, un facteur de croissance, ou une toxine.

12. Formulation pharmaceutique qui comprend en tant qu'ingrédient actif, au moins un anticorps, ou un fragment de celui-ci, selon l'une quelconque des revendications 1 à 6, associé avec un porteur, diluant ou excipient pharmaceutiquement acceptable pour celui-ci.

13. Anticorps monoclonal selon l'une des revendications 1 à 6 qui est un anticorps chimérique ou un fragment de celui-ci.

14. Molécule d'ADNc encodant l'anticorps chimérique ou le fragment de la revendication 13.

## Revendications (Revendications pour l'(les) Etat(s) contractant(s) suivant(s): ES)

1. Procédé pour préparer un anticorps monoclonal, ou fragment de celui-ci, dont le site de combinaison d'antigène se lie à 1' Oncostatine M et inhibe compétitivement la liaison imunospécifique de l'anticorps monoclonal OM2, OM3, ou OM1 produit par les lignes de cellules d'hybridomes 11R2F8 (ATCC HB 10398), 12R13D7 (ATCC HB 10396) et 1R10F11 (ATCC HB 10397), respectivement, à son antigène cible, qui comprend :
(a) la culture d'une ligne de cellules, ladite ligne de cellules capable de produire un anticorps anti-Oncostatine M, ou un fragment de celui-ci, et récupération du produit produit, ou,
(b) la préparation d'un fragment d'anticorps anti-Oncostatine M à partir d'un anticorps anti-Oncostatine M intact ; et si souhaité,
(c) la conjugaison de l'anticorps ou du fragment d'anticorps préparé en (a) ou en (b) à un marqueur, un facteur de croissance, ou une toxine.

2. Procédé selon la revendication 1 qui produit un anticorps monoclonal OM2, OM3, ou OM1 produit par les lignes de cellules d'hybridomes 11R2F8 (ATCC HB 10398), 12R13D7 (ATCC HB 10396) et 1R10F11 (ATCC HB 10397), respectivement, ou un fragment de celui-ci.

3. Procédé selon l'une quelconque des revendications 1 ou 2 dans lequel un fragment Fab, F(ab')₂ ou Fv d'un anticorps monoclonal est produit.

4. Procédé selon l'une quelconque des revendications 1 à 3 dans lequel un anticorps monoclonal, ou un fragment de celui-ci, est conjugué à un marqueur capable de produire un signal détectable.

5. Procédé selon la revendication 4 dans lequel le marqueur comprend un fluoresceur, un radio-marqueur, un chromophore, ou un enzyme.

6. Procédé selon l'une quelconque des revendications 1 à 3 dans lequel l'anticorps monoclonal, ou son fragment, est conjugué à un facteur de croissance ou une toxine.

7. Ligne de cellules capable de produire l'anticorps monoclonal, ou son fragment d'anticorps, tel que défini dans l'une quelconque des revendications 1 à 3.

8. Procédé pour produire les lignes de cellules d'hybridomes 11R2F8 (ATCC HB 10398), 12R13D7 (ATCC HB 10396), ou 1R10F11 (ATCC HB 10397), qui comprend la fusion des lymphocytes d'un hôte mammalien immunisé par l'Oncostatine M avec une cellule de myélome appropriée et la sélection de la cellule d'hybridome.

9. Procédé pour préparer une formulation pharmaceutique qui comprend le mélange d'un anticorps monoclonal, ou de son fragment, tel que défini dans l'une quelconque des revendications 1 à 6, avec un porteur, diluant ou excipient pharmaceutiquement acceptable.

10. Procédé selon la revendication 1 produisant un anticorps chimérique ou un fragment de celui-ci.

11. Procédé de préparation d'une molécule d'ADNc, lequel procédé comprend la fourniture d'une séquence d'ADNc encodant l'anticorps chimérique ou son fragment tel que défini en revendication 10.

## Revendications (Revendications pour l'(les) Etat(s) contractant(s) suivant(s): GR)

1. Anticorps monoclonal, ou fragment de celui-ci, dont le site de combinaison d'antigène se lie à l'Oncostatine M et inhibe compétitivement la liaison imunospécifique de l'anticorps monoclonal OM2, OM3, ou OM1 produit par les lignes de cellules d'hybridomes 11R2F8 (ATCC HB 10398), 12R13D7 (ATCC HB 10396) et 1R10F11 (ATCC HB 10397), respectivement, à son antigène cible.

2. Anticorps monoclonal OM2, OM3, ou OM1 produit par les lignes de cellules d'hybridomes 11R2F8 (ATCC HB 10398), 12R13D7 (ATCC HB 10396) et 1R10F11 (ATCC HB 10397), respectivement, ou fragment de celui-ci.

3. Fragment Fab, F(ab')₂ ou Fv d'un anticorps monoclonal selon l'une quelconque des revendications 1 et 2.

4. Anticorps monoclonal, ou fragment de celui-ci, selon l'une quelconque des revendications 1 à 3, conjugué à un marqueur capable de produire un signal détectable.

5. Anticorps monoclonal, ou fragment de celui-ci, selon la revendication 4, dans lequel le marqueur comprend un fluoresceur, un radio-marqueur, un chromophore, ou un enzyme.

6. Anticorps monoclonal, ou fragment de celui-ci, selon l'une quelconque des revendications 1 à 3, qui est conjugué avec un facteur de croissance ou une toxine.

7. Ligne de cellules capable de produire un anticorps monoclonal, ou un fragment d'anticorps de celui-ci, selon l'une quelconque des revendications 1 à 3.

8. Ligne de cellules d'hybridomes 11R2F8 (ATCC HB 10398), 12R13D7 (ATCC HB 10396), ou 1R10F11 (ATCC HB 10397).

9. Anticorps monoclonal, ou fragment de celui-ci, selon l'une quelconque des revendications 1 à 6, pour utilisation dans le diagnostic ou le traitement d'un état de maladie qui implique l'activité biologique de l'Oncostatine M.

10. Méthode de détection d' Oncostatine M qui comprend la mise en contact d'un échantillon biologique avec un anticorps, ou un fragment de celui-ci, tel que défini dans l'une quelconque des revendications 1 à 6.

11. Procédé pour préparer un anticorps monoclonal tel que défini dans l'une des revendications 1 à 6, où un fragment de celui-ci, dont le site de combinaison d'antigène se lie à l'Oncostatine M, qui comprend :
(a) la culture d'une ligne de cellules, ladite ligne de cellules capable de produire un anticorps anti-Oncostatine M, ou un fragment de celui-ci et la récupération du produit produit ; ou
(b) la préparation d'un fragment d'anticorps anti-Oncostatine M à partir d'un anticorps anti-Oncostatine M intact ; et, si souhaité,
(c) la conjugaison de l'anticorps ou du fragment d'anticorps préparé en (a) ou (b) à un marqueur, un facteur de croissance, ou une toxine.

12. Procédé de préparation d'une formulation pharmaceutique qui comprend le mélange d'un anticorps monoclonal, ou d'un fragment de celui-ci, selon l'une quelconque des revendications 1 à 6, avec un porteur, diluant ou excipient pharmaceutiquement acceptable pour celui-ci.

13. Anticorps monoclonal selon l'une des revendications 1 à 6 qui est un anticorps chimérique ou un fragment de celui-ci.

14. Molécule d'ADNc encodant l'anticorps chimérique ou le fragment de la revendication 13.
